# EUROPÄISCHE PATENTSCHRIFT

(11) **EP 1 660 022 B1**
(45) Veröffentlichungstag und Bekanntmachung des Hinweises auf die Patenterteilung: **12.04.2017**
(21) Anmeldenummer: 04763105.6
(22) Anmeldetag: 06.07.2004
(51) Int. Cl.: A61K 8/18

(54) **MITTEL ZUR FÄRBUNG KERATINISCHER FASERN**
AGENT FOR COLOURING KERATIN FIBRES
AGENT DE COLORATION DES FIBRES KERATINIQUES

(30) Priorität: 04.09.2003 DE 10340695
(43) Veröffentlichungstag der Anmeldung: 31.05.2006
(73) Patentinhaber: HFC Prestige International Holding Switzerland S.a.r.l., 1213 Petit-Lancy (CH)
(72) Erfinder: SCHMENGER, Jürgen, 64331 Weiterstadt (DE); BRAUN, Petra, 64839 Münster (DE); KUJAWA, Jolanthe, 64289 Darmstadt (DE); GLATTFELDER, Martina, 68542 Heddesheim (DE); ENTZMINGER, Anne, 64347 Griesheim (DE); SALLWEY, Anette, D-63303 Dreieich (DE); DEUTZ, Herbert, Woodland Hills, CA 91367 (US)
(74) Vertreter: Vidon Brevets & Stratégie
(86) Internationale Anmeldenummer: PCT/EP2004/007366
(87) Internationale Veröffentlichungsnummer: WO 2005/032502

(56) Entgegenhaltungen:
- EP-A- 0 782 845
- EP-A- 1 166 748
- DE-A- 19 944 527

## Beschreibung

Die vorliegende Erfindung betrifft Zusammensetzungen zur Färbung keratinischer Fasem, insbesonders menschlicher Haare, auf der Basis einer Entwicklersubstanz-Kupplersubstanz-Kombination sowie direktziehenden Farbstoffen, welche eine Färbung der Faser ohne Zusatz eines Oxidationsmittels beständige Färbungen (vergleichbar der Haltbarkeit von semipermanenten Farben) ermöglichen, wobei gleichzeitig eine niedrige Schädigung der Faser (vergleichbar temporären Haarfarben) erfolgt.

Auf dem Gebiet der Färbung von Keratinfasern, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Für weitaus schonendere Anwendungen besteht jedoch ein Bedarf nach Färbemitteln, welche in Gegenwart von Luftsauerstoff auch ohne den Zusatz von Oxidationsmitteln beständige Färbungen ergeben.

Aus der EP 1 166 748 A2 ist es bekannt, dass die Kombination aus Oxidationsfarbstoffen und ggfs. Direktziehern ohne Zusatz von chemischen Oxidationsmitteln zur Haarfärbung Verwendung finden kann; konstante Farbergebnisse (d.h. ein gleichbleibendes Farbbild direkt nach der Anwendung als auch Tage oder Wochen später) ist mit den dort beschriebenen Mitteln nicht möglich.

<DE19944527A1 beschreibt die Verwendung von 2-Nitro-p-Phenylendiaminderivaten der Formel (I), 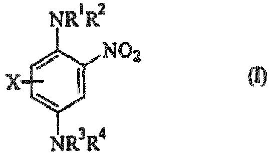 in der R¹ bis R⁴ voneinander unabhängig stehen fur Wasserstoff, eine C₁₋₄-Hydroxyalkylgruppe oder einen gesättigten, einfach oder mehrfach ungesättigten C₅-Ring, der gegebenenfalls mit einer C₁₋₄-Alkylgruppe, einem Halogenatom, einer Hydroxygruppe und/oder einer Aminogruppe substituiert sein kann, und X steht für Wasserstoff oder ein Halogenatom, mit der Maßgabe, daß mindestens einer der Substituenten R₁ bis R₄ ein C₅-Ring ist, als direktziehender Farbstoff zur Verschiebung von Haarfärbungen und -tonungen in den Rotbereich sowie Mittel zum Färben von keratinischen Fasern, die diesen enthalten.>

Aufgabe der vorliegenden Erfindung ist es daher, ein Mittel zur Verfügung zu stellen, welches bel der Luftoxidation die Farbe nicht erst nach Tagen entwickelt, sondern sofort nach der Anwendung eine einheitliche Färbung ermöglicht, die über einen Zeitraum von bis zu 20 Haarwäschen weitest gehend konstant bleibt.

Überraschenderweise wurde nunmehr gefunden, dass durch den Einsatz einer speziellen Kombination von Oxidationsfarbstoffen und direktziehenden Farbstoffen, wobei mindestens 3 direktziehende Farbstoffe enthalten sind und das mengenmäßige Verhältnis von Oxidationsfarbstoffen zu direktziehenden Farbstoffen gleich 5:1 bis 0,5:1 ist, brillante Farbergebnisse erhalten werden, die sich durch einen natürlichen Glanz und eine langanhaltende gleichmäßige Färbung ohne Farbveränderung der Ursprungsfarbe auszeichen. Die Haare werden durch das erfindungsgemäße Mittel sehr schonend gefärbt (vergleichbar den üblicherweise verwendeten leicht auswaschbaren Tönungsmitteln) und praktisch nicht geschädigt. Mit dem erfindungsgemäßen Gegenstand wird ein konstantes Farbbild erreicht, welches Ober einen Zeitraum von bis zu 20 Haarwäschen der gefärbten Faser hält.

Gegenstand der Erfindung ist daher ein Mittel zur Färbung von Keratinfasern, insbesondere Haaren, wie in Anspruch 1 beansprucht. Als geeignete Oxidationsfarbstoffvorstufen können beispielsweise die folgenden Entwicklersubstanzen und Kupplersubstanzen und mit sich selbst kuppelnden Verbindungen genannt werden:
(i) Entwicklersubstanzen: 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diamino-biphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methyl-anilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluor-phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methyl-phenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methylphenyl)methyl]-1 H-pyrazol, 1-[(4-Chlorphenyl)methyl]-4,5-diamino-1H-pyrazol, 4,5-Diamino-1-methyl-1H-pyrazol, 2-Amino-phenol, 2-Amino-6-methyl-phenol, 2-Amino-5-methyl-phenol, allein oder im Gemisch miteinander.
(ii) Kupplersubstanzen: N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxy-ethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxy-ethoxy)-benzol, 1,3-Diamino-4-(2,3-dihydroxypropoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diamino-phenoxy)-methan, 1,3-Diamino-2,4-dimethoxy-benzol, 2,6-Bis(2-hydroxy-ethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)-amino]-2-methyl-phenol, 2-Amino-3-hydroxy-pyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol, 2,3-Indolindion, allein oder im Gemisch miteinander.
(iii) Mit sich selbst kuppelnde Verbindungen: 2-Amino-5-methylphenol, 2-Amino-6-methylphenol, 2-Amino-5-ethoxyphenol oder 2-Propyl-amino-5-aminopyridin.

Unter den vorgenannten Oxidationsfarbstoffen sind die folgenden Verbindungen, alleine oder in Kombination miteinander besonders bevorzugt: 2,5-Diamino-toluol, 2,4-Diaminophenoxyethanol, Resorcin, 2-Methylresorcin, m-Aminophenol, 4-Amino-3-methyl-phenol, 4-Amino-2-hydroxy-toluol, 6-Amino-3-methyl-phenol, 2-Amino-4-hydroxyethylaminoanisol, 1-Naphthol, Hydroxyethyl-3,4-methylendioxyanilin, 2,5-Diamino-phenylethanol, N,N-Bis(2-hydroxyethyl)-p-phenylendiamin, Phenyl-methyl-pyrazolon, und 1-Hydroxyethyl-4,5-diamino-pyrazol oder deren Salze.

Die Gesamtmenge der in dem erfindungsgemäßen Mittel enthaltenen Oxidationsfarbstoffvorstufen beträgt etwa 0,01 bis 12 Gewichtsprozent, insbesondere etwa 0,1 bis 7 Gewichtsprozent. Besonders bevorzugte Farbstoffe sind in Tabelle 1 aufgeführt.

Als direktziehende Farbstoffe können alle üblichen natürlichen und/oder synthetischen direktziehenden Farbstoffe, beispielsweise sogenannte Pflanzenfarbstoffe wie Henna oder Indigo, Triphenylmethanfarbstoffe, aromatische Nitrofarbstoffe, Azofarbstofte, Chinonfarbstoffe, kationische oder anionische Farbstoffe, verwendet werden.

Als geeignete synthetische Farbstoffe können beispielsweise genannt werden: 1,4-Bis[(2-hydroxyethyl)amino]-2-nitrobenzol, 1-(2-Hydroxyethyl)-amino-2-nitro-4-[di(2-hydroxyethyl)-amino]-benzol (HC Blue No. 2), 1-Amino-3-methyl-4-[(2-hydroxyethyl)-amino]-6-nitrobenzol (HC Violet No. 1), 4-[Ethyl-(2-hydroxyethyl)amino]-1-[(2-hydroxyethyl)amino]-2-nitro-benzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxy-propyl)amino]-4-[methyl-(2-hydroxy-ethyl)-amino]-2-nitrobenzol (HC Blue No. 10), 1-[(2,3-Dihydroxypropyl)-amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxy-propyl)amino]-2-nitrobenzol (HC Blue No. 6), 2-((4-Amino-2-nitrophenyl)-amino)-5-dimethylamino-benzoesäure (HC Blue No. 13), 1-Amino-4-[(2-hydroxyethyl)amino]-2-nitrobenzol (HC Red No. 7), 2-Amino-4,6-dinitro-phenol, 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)-amino]-3-nitrophenol, 1-[(2-Aminoethyl)amino]-4-(2-hydroxyethoxy)-2-nitrobenzol (HC Orange No. 2), 4-(2,3-Dihydroxy-propoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino]-2-nitrobenzol (HC Red No. 11), 2-[(2-Hydroxyethyl)amino]-4,6-dinitro-phenol, 4-Ethylamino-3-nitrobenzoesäure, 2-[(4-Amino-2-nitrophenyl)-amino]-benzoesäure, 2-Chlor-6-ethylamino-4-nitrophenol, 2-Amino-6-chlor-4-nitrophenol, 4-[(3-Hydroxy-propyl)amino]-3-nitrophenol, 2,5-Diamino-6-nitropyridin, 1,2,3,4-Tetra-hydro-6-nitrochinoxalin, 7-Amino-3,4-dihydro-6-nitro-2H-1,4-benzoxazin (HC Red No. 14), 1-Amino-2-[(2-hydroxyethyl)amino]-5-nitrobenzol (HC Yellow No. 5), 1-(2-Hydroxy-ethoxy)-2-[(2-hydroxyethyl)-amino]-5-nitrobenzol (HC Yellow No. 4), 1-[(2-Hydroxyethyl)amino]-2-nitrobenzol (HC Yellow No. 2), 2-[(2-Hydroxy-ethyl)-amino]-1-methoxy-5-nitrobenzol, 2-Amino-3-nitrophenol, 1-(2-Hydroxyethoxy)-3-methylamino-4-nitrobenzol, 2,3-(Dihydroxy-propoxy)-3-methyl-amino-4-nitrobenzol, 2-[(2-Hydroxy-ethyl)amino]-5-nitrophenol (HC Yellow No. 11), 3-[(2-Aminoethyl)-amino]-1-methoxy-4-nitrobenzol-hydrochlorid (HC Yellow No.9), 1-[(2-Ureido-ethyl)amino]-4-nitrobenzol, 4-[(2,3-Di-hydroxypropyl)amino]-3-nitro-1-trifluormethyl-benzol (HC Yellow No. 6), 1-Chlor-2,4-bis[(2-hydroxyethyl)-amino]-5-nitrobenzol (HC Yellow No. 10), 4-[(2-Hydroxy-ethyl)amino]-3-nitro-1-methylbenzol, 1-Chlor-4-[(2-hydroxy-ethyl)amino]-3-nitrobenzol (HC Yellow No. 12), 4-[(2-Hydroxyethyl)-amino]-3-nitro-1-trifluormethyl-benzol (HC Yellow No. 13), 4-[(2-Hydroxy-ethyl)amino]-3-nitro-benzonitril (HC Yellow No. 14), 4-[(2-Hydroxyethyl)-amino]-3-nitro-benzamid (HC Yellow No. 15), 1,4-Di[(2,3-dihydroxy-propyl)amino]-9,10-anthrachinon, 1-[(2-Hydroxy-ethyl)amino]-4-methyl-amino-9,10-anthrachinon (CI61505, Disperse Blue No. 3), 2-[(2-Amino-ethyl)amino]-9,10-anthrachinon (HC Orange No. 5), 1-Hydroxy-4-[(4-methyl-2-sulfophenyl)amino]-9,10-anthrachinon, 1-[(3-Aminopropyl)-amino]-4-methylamino-9,10-anthrachinon (HC Blue No. 8),1-[(3-Amino-propyl)-amino]-9,10-anthrachinon (HC Red No. 8), 1,4-Diamino-2-methoxy-9,10-anthrachinon (CI62015, Disperse Red No. 11, Solvent Violet No. 26), 1,4-Dihydroxy-5,8-bis[(2-hydroxyethyl)amino]-9,10-anthrachinon (CI62500, Disperse Blue No. 7, Solvent Blue No. 69), 9-(Dimethylamino)-benzo[a]-phenoxazin-7-ium-chlorid (CI51175; Basic Blue No. 6), Di[4-(diethyl-amino)phenyl][4-(ethylamino)naphthyl]-carbenium-chlorid (CI42595; Basic Blue No. 7), 3,7-Di(dimethylamino)-phenothiazin-5-ium-chlorid (CI52015; Basic Blue No. 9), Di[4-(dimethyl-amino)phenyl][4-(phenylamino)naphthyl]-carbenium-chlorid (CI44045; Basic Blue No. 26), 2-[(4-(Ethyl(2-hydroxy-ethyl)amino)phenyl)azo]-6-methoxy-3-methyl-benzothiazolium-methylsulfat (CI11154; Basic Blue No. 41), 8-Amino-2-brom-5-hydroxy-4-imino-6-[(3-(trimethylammonio)phenyl)-amino]-1(4H)-naphthalinon-chlorid (CI56059; Basic Blue No. 99), Bis[4-(dimethyl-amino)phenyl][4-(methyl-amino)phenyl]carbenium-chlorid (CI42535; Basic Violet No. 1), Tris[4-(dimethylamino)phenyl]carbenium-chlorid (CI42555; Basic Violet No. 3), 2-[3,6-(Diethylamino)-dibenzopyranium-9-yl]-benzoesäure-chlorid (CI45170; Basic Violet No. 10), Di(4-aminophenyl)-(4-amino-3-methyl-phenyl)carbenium-chlorid (CI42510; Basic Violet No. 14), 1,3-Bis[(2,4-diamino-5-methylphenyl)azo]-3-methylbenzol (CI21010; Basic Brown No. 4), 1-[(4-Aminophenyl)azo]-7-(trimethylammonio)-2-naphthol-chlorid (CI12250; Basic Brown No. 16), 1-[(4-Amino-2-nitrophenyl)azo]-7-(trimethylammonio)-2-naphthol-chlorid (Basic Brown No. 17), 1-[(4-Amino-3-nitrophenyl)azo]-7-(trimethyl-ammonio)-2-naphthol-chlorid (CI12251; Basic Brown No. 17), 3,7-Diamino-2,8-dimethyl-5-phenyl-phenazinium-chlorid (CI50240; Basic Red No. 2), 1,4-Dimethyl-5-[(4-(dimethylamino)phenyl)-azo]-1,2,4-triazolium-chlorid (CI11055; Basic Red No. 22), 2-Hydroxy-1-[(2-methoxy-phenyl)azo]-7-(trimethylammonio)-naphthalin-chlorid (CI12245; Basic Red No. 76), 2-[2-((2,4-Dimethoxyphenyl)amino)ethenyl]-1,3,3-trimethyl-3H-indol-1-ium-chlorid (CI48055; Basic Yellow No. 11), 3-Methyl-1-phenyl-4-[(3-(trimethylammonio)-phenyl)azo]-pyrazol-5-on-chlorid (CI12719; Basic Yellow No. 57), Bis[4-(diethylamino)phenyl]phenylcarbenium-hydrogensulfat(1:1) (CI42040; Basic Green No. 1), 1-[Di(2-hydroxyethyl)-amino]-3-methyl-4-[(4-nitro-phenyl)azo]-benzol (CI11210, Disperse Red No. 17), 4-[(4-Aminophenyl)-azo]-1-[di(2-hydroxyethyl)amino]-3-methyl-benzol (HC Yellow No. 7), 2,6-Diamino-3-[(pyridin-3-yl)azo]-pyridin, 6-Hydroxy-5-[(4-sulfophenyl)azo]-2-naphthalinsulfonsäure-dinatriumsalz (CI15985; Food Yellow No. 3; FD&C Yellow No. 6), 2,4-Dinitro-1-naphthol-7-sulfonsäure-dinatriumsalz (CI10316; Acid Yellow No. 1; Food Yellow No. 1), 2-(Indan-1,3-dion-2-yl)chinolin-x,x-sulfonsäure (Gemisch aus Mono- und Disulfonsäure) (CI47005;D&C Yellow No. 10; Food Yellow No. 13; Acid Yellow No. 3), 5-Hydroxy-1-(4-sulfophenyl)-4-[(4-sulfophenyl)-azo]pyrazol-3-carbonsäure-trinatriumsalz (CI19140; Food Yellow No. 4; Acid Yellow No. 23), 9-(2-Carboxyphenyl)-6-hydroxy-3H-xanthen-3-on (CI45350; Acid Yellow No. 73; D&C Yellow No. 8), 5-[(2,4-Dinitrophenyl)-amino]-2-phenylamino-benzolsulfonsäure-natriumsalz (CI10385; Acid Orange No. 3), 4-[(2,4-Dihydroxyphenyl)azo]-benzolsulfonsäure-mononatriumsalz (CI14270; Acid Orange No. 6), 4-[(2-Hydroxynaphth-1-yl)azo]-benzolsulfonsäure-natriumsalz (CI15510; Acid Orange No. 7), 4-[(2,4-Dihydroxy-3-[(2,4-dimethylphenyl)azo]phenyl)azo]-benzolsulfonsäure-natriumsalz (CI20170; Acid Orange No. 24), 4-Hydroxy-3-[(4-sulfonaphth-1-yl)azo]-1-naphthalin-sulfonsäure-dinatriumsalz (CI14720; Acid Red No. 14), 6-Hydroxy-5-[(4-sulfonaphth-1-yl)azo]-2, 4-naphthalin-disulfonsäure-trinatriumsalz (CI16255; Ponceau 4R; Acid Red No. 18), 3-Hydroxy-4-[(4-sulfonaphth-1-yl)azo]-2,7-naphthalin-disulfonsäure-trinatriumsalz (CI16185; Acid Red No. 27), 8-Amino-1-hydroxy-2-(phenylazo)-3,6-naphthalin-disulfonsäure-dinatriumsalz (CI17200; Acid Red No. 33), 5-(Acetylamino)-4-hydroxy-3-[(2-methylphenyl)azo]-2,7-naphthalin-disulfonsäure-dinatriumsalz (CI18065; Acid Red No. 35), 2-(3-Hydroxy-2,4,5,7-tetraiod-dibenzopyran-6-on-9-yl)-benzoesäure-dinatriumsalz (CI45430;Acid Red No. 51), N-[6-(Diethylamino)-9-(2,4-disulfophenyl)-3H-xanthen-3-yliden]-N-ethylethanammonium-hydroxid, inneres Salz, Natriumsalz (CI45100; Acid Red No. 52), 8-[(4-(Phenylazo)-phenyl)azo]-7-naphthol-1,3-disulfonsäure-dinatriumsalz (CI27290; Acid Red No. 73), 2',4',5',7'-Tetrabrom-3',6'-dihydroxyspiro-[isobenzofuran-1 (3H),9'-[9H]xanthen]-3-on-dinatriumsalz (CI45380; Acid Red No. 87), 2',4',5',7'-Tetrabrom-4,5,6,7-tetrachlor-3',6'-dihydroxyspiro[isobenzofuran-1(3H),9'[9H]xanthen]-3-on-dinatriumsalz (CI45410; Acid Red No. 92), 3',6'-Dihydroxy-4',5'-diiodospiro[isobenzofuran-1(3H),9'(9H)-xanthen]-3-on-dinatriumsalz (CI45425; Acid Red No. 95), (2-Sulfophenyl)di[4-(ethyl((4-sulfophenyl)methyl)amino)phenyl]-carbenium-dinatriumsalz, betain (CI42090; Acid Blue No. 9; FD&C Blue No. 1), 1,4-Bis[(2-sulfo-4-methylphenyl)amino]-9,10-anthrachinon-dinatriumsalz (CI 61570; Acid Green No. 25), Bis[4-(dimethylamino)phenyl]-(3,7-disulfo-2-hydroxy-naphth-1-yl)carbenium-inneres Salz, mononatriumsalz (CI44090; Food Green No. 4; Acid Green No. 50), Bis[4-(diethylamino)phenyl](2,4-disulfophenyl)carbenium-inneres salz, Natriumsalz (2:1) (CI42045; Food Blue No. 3; Acid Blue No. 1), Bis[4-(diethylamino)phenyl](5-hydroxy-2,4-disulfophenyl)carbenium-inneres salz, Calciumsalz (2:1) (CI42051; Acid Blue No. 3), 1-Amino-4-(cyclohexylamino)-9,10-anthrachinon-2-sulfon-säure-natriumsalz (CI62045; Acid Blue No. 62),2-(1,3-Dihydro-3-oxo-5-sulfo-2H-indol-2-yliden)-2,3-dihydro-3-oxo-1H-indol-5-sulfonsäure-dinatriumsalz (CI73015; Acid Blue No. 74), 9-(2-Carboxyphenyl)-3-[(2-methylphenyl)amino]-6-[(2-methyl-4-sulfophenyl)amino]xanthylium-inneres Salz, mononatriumsalz (CI45190; Acid Violet No. 9), 1-Hydroxy-4-[(4-methyl-2-sulfophenyl)amino]-9,10-anthrachinon-natriumsalz (CI60730; D&C Violett No. 2; Acid Violet No. 43), Bis[3-nitro-4-[(4-phenylamino)-3-sulfo-phenylamino]-phenyl]-sulfon (CI10410; Acid Brown No. 13), 5-Amino-4-hydroxy-6-[(4-nitrophenyl)azo]-3-(phenylazo)-2,7-naphthalindisulfonsäure-dinatriumsalz (CI20470; Acid Black No. 1), 3-Hydroxy-4-[(2-hydroxynaphth-1-yl)azo]-7-nitro-1-naphthalin-sulfonsäure-chromkomplex (3:2) (CI15711; Acid Black No. 52), 3-[(2,4-Dimethyl-5-sulfophenyl)azo]-4-hydroxy-1-naphthalin-sulfonsäure-dinatriumsalz (CI14700; Food Red No. 1; FD&C Red No. 4), 4-(Acetylamino)-5-hydroxy-6-[(7-sulfo-4-[(4-sulfophenyl)azo]naphth-1-yl)azo]-1,7-naphthalindisulfonsäure-tetranatriumsalz (CI28440; Food Black No. 1) und 3-Hydroxy-4-(3-methyl-5-oxo-1-phenyl-4,5-dihydro-1H-pyrazol-4-ylazo)-naphthalin-1-sulfonsäure-natriumsalz, Chrom-Komplex (Acid Red No. 195), alleine oder in Kombination miteinander.

Unter den vorgenannten direktziehenden Farbstoffen sind die folgenden Verbindungen -alleine oder in Kombination miteinander- besonders bevorzugt: Hydroxyethyl-2-nitro-p-toluidin, 2-Hydroxyethyl-pikraminsäure, 4-Nitrophenyl-aminoharnstoff, Tri(4-amino-3-methylphenyl)-carbenium-chlorid (Basic Violet 2), 1,4-Diamino-9,10-anthracendion (Disperse Violet 1), 1-(2-Hydroxyethyl)-amino-2-nitro-4-[di(2-hydroxyethyl)-amino]-benzol (HC Blue No. 2), 4-[Ethyl-(2-hydroxyethyl)amino]-1-[(2-hydroxyethyl)-amino]-2-nitro-benzol-hydrochlorid (HC Blue No. 12), 1-Amino-4-[di(2-hydroxyethyl)amino]-2-nitrobenzol-hydrochlorid (HC Red No. 13), 3-((2-Nitro-4-(trifluoromethyl)phenyl)amino)-1,2-propandiol (HC Yellow No. 6), 4-Amino-1-[(2-hydroxyethyl)amino]-2-nitrobenzol (HC Red No. 3), 4-Amino-3-nitrophenol, 4-[(2-Hydroxyethyl)-amino]-3-nitrophenol, 1-Amino-5-chlor-4-[(2,3-dihydroxypropyl)amino]-2-nitrobenzol (HC Red No. 10), 5-Chlor-1,4-[di(2,3-dihydroxypropyl)amino]-2-nitrobenzol (HC Red No. 11), 2-Chlor-6-ethylamino-4-nitrophenol, 2-Amino-6-chlor-4-nitrophenol, 4-[(2-Hydroxyethyl)-amino]-3-nitro-1-trifluormethyl-benzol (HC Yellow No. 13), 8-Amino-2-brom-5-hydroxy-4-imino-6-[(3-(trimethylammonio)phenyl)-amino]-1(4H)-naphthalinon-chlorid (CI56059; Basic Blue No. 99), 1-[(4-Aminophenyl)azo]-7-(trimethylammonio)-2-naphthol-chlorid (CI12250; Basic Brown No. 16), 1-[(4-Amino-2-nitrophenyl)azo]-7-(trimethylammonio)-2-naphthol-chlorid (Basic Brown No. 17), 2-Hydroxy-1-[(2-methoxy-phenyl)azo]-7-(trimethylammonio)-naphthalin-chlorid (CI12245; Basic Red No. 76), 3-Methyl-1-phenyl-4-[(3-(trimethylammonio)-phenyl)azo]-pyrazol-5-on-chlorid (CI12719; Basic Yellow No. 57) und 2,6-Diamino-3-[(pyridin-3-yl)azo]-pyridin sowie deren Salze.

Die Gesamtmenge der direktziehenden Farbstoffe beträgt in dem erfindungsgemässen Mittel etwa 0,01 bis 7 Gewichtsprozent, vorzugsweise etwa 0,2 bis 4 Gewichtsprozent. Besonders bevorzugte Farbstoffe sind in Tabelle 1 aufgeführt.

Weitere zur Haarfärbung bekannte und übliche Farbstoffe, die in dem erfindungsgemässen Färbemittel enthalten sein können, sind unter anderem in E. Sagarin, "Cosmetics, Science and Technology", Inter-science Publishers Inc., New York (1957), Seiten 503 ff. sowie H. Janistyn, "Handbuch der Kosmetika und Riechstoffe", Band 3 (1973), Seiten 388 ff. und K. Schrader "Grundlagen und Rezepturen der Kosmetika", 2. Auflage (1989), Seiten 782-815 beschrieben, auf die hier-mit ausdrücklich bezug genommen wird.

Darüberhinaus können in dem erfindungsgemäßen Mittel Antioxidantien wie zum Beispiel Ascorbinsäure, Thioglykolsäure oder Natriumsulfit, sowie Komplexbildner für Schwermetalle, beispielsweise Ethylendiaminotetraacetat oder Nitriloessigsäure, in einer Menge von bis zu etwa 0,5 Gewichtsprozent enthalten sein. Parfümöle können in der erfindungsgemäßen Farbträgermasse in einer Menge von bis zu etwa 1 Gewichtsprozent enthalten sein. Selbstverständlich kann das vorstehend beschriebene Haarfärbemittel gegebenenfalls weitere, für Haarfärbemittel übliche Zusätze, wie zum Beispiel Verdickungsmittel, beispielsweise Homopolymere der Acrylsäure, Pflanzen Gums, Cellulose- und Stärkederivate, Algenpolyasaccharide, amphiphile Assoziatiwerdicker, desweiteren Konservierungsstoffe; Komplexbildner; Lösungsmittel wie Wasser, niedere aliphatische Alkohole, beispielsweise aliphatische Alkohole mit 1 bis 4 Kohlenstoffatomen wie Ethanol, Propanol und Isopropanol, oder Glykole wie Glycerin und 1,2-Propylenglykol; Netzmittel oder Emulgatoren, aus den Klassen der anionischen, kationischen, amphoteren oder nichtionogenen oberflächenaktiven Substanzen; weiterhin Weichmacher; Vaseline; Silikonöle, Paraffinöl, Polysorbate und Fett-säuren sowie außerdem Pflegestoffe, wie kationische Polymere oder Harze, Lanolinderivate, Cholesterin, Vitamine, Pantothensäure und Betain, enthalten. Die erwähnten Bestandteile werden in den für solche Zwecke üblichen Mengen verwendet, zum Beispiel die Netzmittel und Emulgatoren in Konzentrationen von 0,1 bis 30 Gewichtsprozent und die Pflegestoffe in einer Konzentration von 0,1 bis 5,0 Gewichtsprozent.

Der pH-Wert des erfindungsgemäßen Färbemittels liegt im Bereich von etwa 5 bis 11, vorzugsweise 7 bis 9.

Je nach Zusammensetzung und gewünschtem pH-Wert des Färbemittels erfolgt die Einstellung des pH-Wertes vorzugsweise mit organischen Aminen, wie zum Beispiel Glucaminen, Aminomethylpropanol, Monoethanolamin oder Triethanolamin, es können aber auch Ammoniak oder anorganischen Basen, beispielsweise Natriumhydroxid, Kaliumhydroxid, Natriumcarbonat oder Calciumhydroxid, beziehungsweise organische oder anorganische Säuren, wie zum Beispiel Milchsäure, Zitronensäure, Essigsäure oder Phosphorsäure Verwendung finden.

Das erfindungsgemäße Mittel wird auf die trockene oder vorher gewaschene Faser aufgetragen und nach einer Einwirkungszeit von 5 bis 60 Minuten, vorzugsweise nach 10 bis 45 Minuten, mit Wasser ausge-spült und ggfs. mit einem Shampoo gewaschen. Die Keratinfaser kann ggfs. in üblicher Weise nachbehandelt und getrocknet werden.

Ein weiterer Gegenstand ist die Verwendung einer wässrigen oder wässrig-alkoholischen Zubereitung, wie in Anspruch 5 beansprucht.

Die nachfolgenden Beispiele sollen den Gegenstand näher erläutern, ohne ihn auf diese Beispiele zu beschränken:

**Beispiel 1: Cremeförmiges Aerosolfärbemittel**

| | |
|---|---|
| 1,2 g | Cetylstearylalkohol |
| 0,6 g | Natriumlaurylsulfat |
| 0,1 g | Ethylendiaminotetraessigsäuredinatriumsalz |
| 0,2 g | Ascorbinsäure |
| 2,5 g | 1,2 Propylenglykol |
| 2,5 g | Ethanol |
| 4,5 g | Ammoniak |
| 2,5 g | Monoethanolamin |
| 1,3 g | 2,5-Diamino-toluol-sulfat |
| 0,7 g | 5-Amino-2-methyl-phenol |
| 1,3 g | 2,2'-((4-((2-hydroxyethal)amino)-3-nitrophenyl)imino)bis-ethanol (HC Blue No.2) |
| 0,5 g | 2-((4-Amino-2-nitrophenyl)amino)-ethanol (HC Red No. 3) |
| 0,6 g | 3-(4-Amino-2-chloro-5-nitrophenyl)amino-1,2 propandiol (HC Red No. 10 + 11) |
| 0,3 g | Parfümöl |
| ad 100,0 g | Wasser |

Die im Heißemulgierverfahren hergestellten Farbstoffmassen werden in einem geeigneten Druckgasbehälter mit Propan/Butan und F 152 (Verhältnis 1:1) im Verhältnis Wirkstoff/Treibgas 95:5 % abgefüllt.
Der Aerosolschaum wird auf die trockenen Haare aufgetragen und nach einer Einwirkungszeit von 20 Minuten mit Wasser ausgespült. Das Haar wird sodann getrocknet. Es wird eine glänzende, rotbraune Färbung erhalten.

**Beispiel 2: Gelförmiges Aerosolfärbemittel**

| | |
|---|---|
| 2,00 g | Hydroxyethylcellulose |
| 20,00 g | Cocamidopropylbetain |
| 1,50 g | D-Panthenol |
| 10,00 g | Ethanol |
| 0,10 g | Dinatriumtetraborat |
| 0,10 g | Ethylendiaminotetraessigsäuredinatriumsalz |
| 0,20 g | Ascorbinsäure |
| 4,50 g | Monoethanolamin |
| 2,00 g | 2,5-Diamino-toluol-sulfat |
| 0,75 g | Resorcin |
| 0,70 g | 2-Amino-4-(2'-hydroxyethylamino)-anisol |
| 0,35 g | 3-Aminophenol |
| 3,10 g | (4-(Ethyl((2-hydroxyethyl)amino)-2-nitrophenyl)amino)-ethanol-hydrochlorid (HC Blue No. 12) |
| 1,00 g | 3-((2-Nitro-4-(trifluoromethyl)phenyl)amino)-1,2-propandiol (HC Yellow No. 6) |
| 1,00 g | 3-((4,5-Dihydro-3-methyl-5-oxo-1-phenyl-1H-pyrazol-4-yl)azo)-N,N,N-trimethyl-benzenaminiumchlorid (Basic Yellow No. 57) |
| 0,47 g | 1,4-Diamino-9,10-anthracendion (Disperse Violet No. 1) |
| 0,70 g | 1-N-Hydroxyethylamino-4-methyl-2-nitro-benzol |
| 3,10 g | Natronlauge, 20%ige wässrige Lösung |
| 0,50 g | Parfümöl |
| ad 100,00 g | Wasser |

Die im Kaltmischverfahren hergestellte Gelgrundlage wird mit den heißgelösten Farbstoffen vereinigt und in einem geeigneten Druckgasbehälter mit Propan/Butan und F 152 (Verhältnis 1:1) im Verhältnis Wirk-stoff/Treibgas 95:5 % abgefüllt.
Der Aerosolschaum wird auf die gewaschenen Haare aufgetragen und nach einer Einwirkungszeit von 15 Minuten mit Wasser ausgespült. Das Haar wird sodann mit einem milden Shampoo gewaschen, erneut mit Wasser gespült und anschließend getrocknet. Es wird eine glänzende, dunkelbraune Färbung erhalten.

**Beispiel 3: Gelförmiges kationisches Aerosolfärbemittel**

| | |
|---|---|
| 1,300 g | Hydroxyethylcellulose |
| 6,000 g | Cocamidopropylbetain |
| 2,000 g | Polyethylenglykol300caprylglycerid (Softigen® 767) |
| 3,000 g | Decylglucosid (Plantaren® 2000) |
| 1,500 g | Cetylrimethylammoniumchlorid |
| 5,000 g | Isopropanol |
| 0,200 g | Ascorbinsäure |
| 0,100 g | Ethylendiaminotetraessigsäuredinatriumsalz |
| 2,000 g | 2,5-Diamino-toluol-sulfat |
| 0,750 g | Resorcin |
| 0,700 g | 2-Amino-4-(2'-hydroxyethylamino)-anisol |
| 0,350 g | 3-Aminophenol |
| 5,800 g | Monoethanolamin |
| 0,300 g | (4-(Ethyl((2-hydroxyethyl)amino)-2-nitrophenyl)amino)-ethanol-hydrochlorid (HC Blue No. 12) |
| 1,000 g | 2,2'-((4-((2-hydroxyethal)amino)-3-nitrophenyl)imino)bis-ethanol (HC Blue No. 2) |
| 0,400 g | 3-((2-Nitro-4-(trifluoromethyl)phenyl)amino)-1,2-propandiol (HC Yellow No.6) |
| 0,013 g | 2,6-Diamino-3-((pyridin-3-yl)azo)pyridin |
| 0,170 g | 3-Hydroxy-4-((2-hydroxy-1-naphthalinyl)azo)-7-nitro-1-naphthalinsulfonsäure-mononatriumsal (Acid Black No. 52) |
| 0,066 g | 3-(4-Amino-2-chloro-5-nitrophenyl)amino-1,2 propandiol (HC Red No. 10 + 11) |
| 0,500 g | Parfümöl |
| ad 100,000 g | Wasser |

Die im Kaltmischverfahren hergestellte Gelgrundlage wird mit den heißgelösten Farbstoffen vereinigt und in einem geeigneten Druckgasbehälter mit Propan/Butan und F 152 (Verhältnis 1:1) im Verhältnis Wirk-stoff/Treibgas 95:5 % abgefüllt. Der Aerosolschaum wird auf die trockenen Haare aufgetragen und nach einer Einwirkungszeit von 30 Minuten mit Wasser ausgespült. Das Haar wird sodann getrocknet. Es wird eine glänzende, schwarze Färbung erhalten.

**Beispiel 4: Flüssiges Luftoxidationsfärbemittel**

| | |
|---|---|
| 2,50 g | Oleinsäure |
| 2,00 g | Cetearylalkohol |
| 4,00 g | Undeceth-3 |
| 5,00 g | Ethanol |
| 2,50 g | Aminomethylpropanol |
| 0,43 g | 2,5-Diamino-toluol-sulfat |
| 0,47 g | Resorcin |
| 0,31 g | 2-Methyl-resorcin |
| 1,59 g | 4-Amino-3-methyl-phenol |
| 1,01 g | 5-Amino-2-methyl-phenol |
| 0,30 g | 2,2'-((4-((2-hydroxyethal)amino)-3-nitrophenyl)imino)bis-ethanol (HC Blue No. 2) |
| 0,30 g | 3-(4-Amino-2-chloro-5-nitrophenyl)amino-1,2 propandiol (HC Red No. 10 + 11) |
| 0,20 g | 2-Amino-6-chlor-4-nitro-phenol |
| ad 100,00 g | Wasser |

Das Färbemittel wird auf die trockenen Haare aufgetragen und nach einer Einwirkungszeit von 20 Minuten mit Wasser ausgespült. Das Haar wird sodann getrocknet. es wird eine glänzende, granatrote Färbung erhalten.

Alle Prozentangaben stellen soweit nicht anders angegeben, Gewichtsprozente dar.

## Patentansprüche

1. Mittel zur Färbung von Keratinfasern, welches frei von chemischen Oxidationsmitteln ist, **dadurch gekennzeichnet, dass** es in Form einer wässrigen oder wässrig-alkoholischen Zubereitung vorliegt und eine Kombination von Oxidationsfarbstoffen und direktziehenden Farbstoffen enthält, wobei mindestens 3 direktziehende Farbstoffe enthalten sind und das gewichtsmäßige Verhältnis von Oxidationsfarbstoffen zu direktziehenden Farbstoffen gleich 5:1 bis 0,5:1 ist und dass die direktziehenden Farbstoffe ausgewählt sind aus Hydroxyethyl-2-nitro-p-toluidin, 2-Hydroxyethyl-pikraminsäure, 4-Nitrophenyl-aminohamstoff, Basic Violet 2, Disperse Violet 1, HC Blue No. 2, HC Blue No. 12, HC Red No. 13, HC YellowNo. 6, HC Red No. 3, 4-Amino-3-nitrophenol, 4-[(2-Hydroxyethyl)-amino]-3-nitrophenol, HC Red No. 10, HC Red No. 11 , 2-Chlor-6-ethylamino-4-nitrophenol, 2-Amino-6-chlor-4-nitrophenol, HC Yellow No. 13, Basic Blue No. 99, Basic Brown No. 16, Basic Brown No. 17, Basic Red No. 76, Basic Yellow No. 57 und 2,6-Diamino-3-[(pyridin-3-yl)azo]-pyridin sowie deren Salzen und dass die Oxidationsfarbstoffe ausgewählt sind aus 2,5-Diamino-toluol, 2,4-Diaminophenoxyethanol, Resorcin, 2-Methylresorcin, m-Aminophenol, 4-Amino-3-methyl-phenol, 4-Amino-2-hydroxy-toluol, 6-Amino-3-methyl-phenol, 2-Amino-4-hydroxyethylaminoanisol, 1-Naphthol, Hydroxyethyl-3,4-methylendioxyanilin, 2,5-Diamino-phenylethanol, N,N-Bis(2-hydroxyethyl)-p-phenylendiamin, Phenyl-methyl-pyrazolon und 1-Hydroxyethyl-4,5-diamino-pyrazol oder deren Salzen.

2. Mittel nach Anspruch 1, **dadurch gekennzeichnet, dass** es ein Haarfärbemittel ist.

3. Verfahren zum Färben von Keratinfasern, **dadurch gekennzeichnet, dass** ein Mittel nach einem der Ansprüche 1 bis 2 auf die Faser aufgetragen und nach einer Einwirkungszeit von 5 bis 60 Minuten mit Wasser ausgespült wird und die Faser abschließend getrocknet wird.

4. Verfahren nach Anspruch 3, **dadurch gekennzeichnet, dass** die Faser vor dem Trocknen mit einem Shampoo gewaschen und sodann mit Wasser gespült wird.

5. Verwendung einer wässrigen oder wässrig-alkoholischen Zubereitung, welche eine Kombination von Oxidationsfarbstoffen und direktziehenden Farbstoffen enthält, wobei mindestens 3 direktziehende Farbstoffe enthalten sind und das gewichtsmäßige Verhältnis von Oxidationsfarbstoffen zu direktziehenden Farbstoffen gleich 5:1 bis 0,5:1 ist, zur Herstellung eine Mittels zur Färbung von Keratinfasern, welches frei von chemischen Oxidationsmitteln ist und bei der Luftoxidation sofort nach der Anwendung eine einheitliche und stabile Färbung ergibt und wobei die direktziehenden Farbstoffe aus Hydroxyethyl-2-nitro-p-toluidin, 2-Hydroxyethyl-pikraminsäure, 4-Nitrophenyl-aminohamstoff, Basic Violet 2, Disperse Violet 1, HC Blue No. 2, HC Blue No. 12, HC Red No. 13, HC Yellow No. 6, HC Red No. 3, 4-Amino-3-nitrophenol, 4-[(2-Hydroxyethyl)-amino]-3-nitrophenol, HC Red No. 10, HC Red No. 11, 2-Chlor-6-ethylamino-4-nitrophenol, 2-Amino-6-chlor-4-nitrophenol, HC Yellow No. 13, Basic Blue No. 99, Basic Brown No. 16, Basic Brown No. 17, Basic Red No. 76, Basic Yellow No. 57 und 2,6-Diamino-3-[(pyridin-3-yl)azo]-pyridin sowie deren Salzen ausgewählt sind und wobei die Oxidationsfarbstoffe aus 2,5-Diamino-toluol, 2,4-Diaminophenoxyethanol, Resorcin, 2-Methylresorcin, m-Aminophenol, 4-Amino-3-methyl-phenol, 4-Amino-2-hydroxy-toluol, 6-Amino-3-methyl-phenol, 2-Amino-4-hydroxyethylaminoanisol, 1-Naphthol, Hydroxyethyl-3,4-methylendioxyanilin, 2,5-Diamino-phenylethanol, N,N-Bis(2-hydroxyethyl)-p-phenylendiamin, Phenyl-methyl-pyrazolon und 1-Hydroxyethyl-4,5-diamino-pyrazol oder deren Salzen ausgewählt sind.

## Claims

1. An agent for dying keratin fibres, which is free of chemical oxidising agents, **characterised in that** it is present in the form of an aqueous or aqueous-alcoholic preparation and comprises a combination of oxidation dyes and direct dyes, which comprises at least 3 direct dyes and the weight ratio between oxidation dyes and direct dyes is from 5:1 to 0.5:1, and **in that** the direct dyes are selected from hydroxyethyl-2-nitro-p-toluidine, 2-hydroxyethyl-picramic acid,4-nitrophenyl-amino urea, Basic Violet 2, Disperse Violet 1, HC Blue No. 2, HC Blue No. 12, HC Red No. 13, HC Yellow No. 6, HC Red No. 3, 4-amino-3-nitrophenol, 4-[(2-hydroxyethyl)-amino]-3-nitrophenol, HC Red No. 10, HC Red No. 11, 2-chloro-6-ethylamino-4-nitrophenol, 2-amino-6-chloro-4-nitrophenol, HC Yellow No. 13, Basic Blue No. 99, Basic Brown No. 16, Basic Brown No. 17, Basic Red No. 76, Basic Yellow No. 57 and 2,6-diamino-3-[(pyridin-3-yl)azo]-pyridine, as well as the salts thereof, and **in that** the oxidation dyes are selected from 2,5-diamino-toluene, 2,4-diaminophenoxyethanol, resorcin, 2-methylresorcin, m-aminophenol, 4-amino-3-methyl-phenol, 4-amino-2-hydroxy-toluene, 6-amino-3-methyl-phenol, 2-amino-4-hydroxyethylaminoanisole, 1-naphthol, hydroxyethyl-3,4-methylenedioxyanilin, 2,5-diamino-phenylethanol, N,N-bis(2-hydroxyethyl)-p-phenylene diamine, phenyl-methyl-pyrazolone and 1-hydroxyethyl-4,5-diamino-pyrazole or the salts thereof.

2. The agent as claimed in claim 1, **characterised in that** it is a hair dyeing agent.

3. A method for dyeing keratin fibres, characterised that an agent as claimed in any one of claims 1 to 2 is applied to the fibres and is rinsed off with water after a soaking time of 5 to 60 minutes and the fibres are subsequently dried.

4. The method as claimed in claim 3, **characterised in that** the fibres are washed with a shampoo prior to drying and are then rinsed with water.

5. The use of an aqueous or aqueous-alcoholic preparation, which comprises a combination of oxidation dyes and direct dyes, which comprises at least 3 direct dyes and the weight ratio between oxidation dyes and direct dyes is from 5:1 to 0.5:1, for the production of an agent for dyeing keratin fibres, which is free of chemical oxidation agents and upon air oxidation immediately after application results in a consistent and stable colouring, and wherein the direct dyes are selected from hydroxyethyl-2-nitro-p-toluidine, 2-hydroxyethyl-picramic acid, 4-nitrophenyl-amino urea, Basic Violet 2, Disperse Violet 1, HC Blue No. 2, HC Blue No. 12, HC Red No. 13, HC Yellow No. 6, HC Red No. 3, 4-amino-3-nitrophenol, 4-[(2-hydroxyethyl)-amino]-3-nitrophenol, HC Red No. 10, HC Red No. 11, 2-chloro-6-ethylamino-4-nitrophenol, 2-amino-6-chloro-4-nitrophenol, HC Yellow No. 13, Basic Blue No. 99, Basic Brown No. 16, Basic Brown No. 17, Basic Red No. 76, Basic Yellow No. 57 and 2,6-diamino-3-[(pyridin-3-yl)azo]-pyridine, as well as the salts thereof, and that the oxidation dyes are selected from 2,5-diamino-toluene, 2,4-diaminophenoxyethanol, resorcin, 2-methylresorcin, m-aminophenol, 4-amino-3-methyl-phenol, 4-amino-2-hydroxy-toluene, 6-amino-3-methyl-phenol, 2-amino-4-hydroxyethylaminoanisole, 1-naphthol, hydroxyethyl-3,4-methylenedioxyanilin, 2,5-diamino-phenylethanol, N,N-bis(2-hydroxyethyl)-p-phenylenediamine, phenyl-methyl-pyrazolone and 1-hydroxyethyl-4,5-diamino-pyrazole or the salts thereof.

## Revendications

1. Agent de coloration de fibres kératiniques, dépourvu d'agents oxydants chimiques, **caractérisé en ce qu'**il se présente sous la forme d'une préparation aqueuse ou hydro-alcoolique et comprend une combinaison de colorants par d'oxydation et de colorants directs, dans lequel au moins 3 colorants directs sont contenus et le rapport en poids de colorants par oxydation par rapport aux colorants directs est de 5:1 à 0,5:1 et **en ce que** les colorants directs sont choisis parmi l'hydroxyéthyl-2-nitro-p-toluidine, l'acide 2-hydroxyéhyl-picramique, la 4-nitrophényl-amino-urée, le Basic Violet 2, le Disperse Violet 1, le HC Blue n° 2, le HC Blue n° 12, le HC Red n° 13, le HC Yellow n° 6, le HC Red n° 3, le 4-amino-3-nitrophénol, le 4-[(2-hydroxyéthyl)-amino]-3-nitrophénol, le HC Red n° 10, le HC Red n° 11, le 2-chloro-6-éthylamino-4-nitrophénol, le 2-amino-6-chloro-4-nitrophénol, le HC Yellow n° 13, le Basic Blue n° 99, le Basic Brown n° 16, le Basic Brown n° 17, le Basic Red n° 76, le Basic Yellow n° 57 et la 2,6-diamino-3-[(pyridin-3-yl)azo]-pyridine ainsi que leurs sels, et **en ce que** les colorants par oxydation sont choisis parmi le 2,5-diamino-toluène, le 2,4-diaminophénoxyéthanol, la résorcine, la 2-méthylrésorcine, le m-aminophénol, le 4-amino-3-méthyl-phénol, le 4-amino-2-hydroxy-toluène, le 6-amino-3-méthyl-phénol, le 2-amino-4-hydroxyéthylaminoanisol, le 1-naphtol, l'hydroxyéthyl-3,4-méthylènedioxyaniline, le 2,5-diamino-phényléthanol, la N,N-bis(2-hydroxyéthyl)-p-phénylène-diamine, la phényl-méthyl-pyrazolone et le 1-hydroxyéthyl-4,5-diamino-pyrazol ou leurs sels.

2. Agent selon la revendication 1, **caractérisé en ce qu'**il s'agit d'un agent de coloration capillaire.

3. Procédé de coloration de fibres kératiniques, **caractérisé en ce qu'**un agent selon l'une des revendications 1 à 2 est appliqué sur les fibres et, après un temps d'action de 5 à 60 minutes, est rincé à l'eau et les fibres sont finalement séchées.

4. Procédé selon la revendication 3, **caractérisé en ce que**, avant le séchage, les fibres sont lavées avec un shampooing et ensuite rincée à l'eau.

5. Utilisation d'une préparation aqueuse ou hydro-alcoolique qui comprend une combinaison de colorants par oxydation et de colorants directs, dans laquelle au moins 3 colorants directs sont contenus et le rapport en poids de colorants par oxydation par rapport aux colorants directs est de 5:1 à 0,5:1, pour la production d'un agent de coloration de fibres kératiniques dépourvu d'agents oxydants chimiques et qui donne lors de l'oxydation à l'air immédiatement après l'utilisation, une coloration uniforme et stable, et dans laquelle les colorants directs sont choisis parmi l'hydroxyéthyl-2-nitro-p-toluidine, l'acide 2-hydroxyéhyl-picramique, la 4-nitrophényl-amino-urée, le Basic Violet 2, le Disperse Violet 1, le HC Blue n° 2, le HC Blue n° 12, le HC Red n° 13, le HC Yellow n° 6, le HC Red n° 3, le 4-amino-3-nitrophénol, le 4-[(2-hydroxyéthyl)-amino]-3-nitrophénol, le HC Red n° 10, le HC Red n° 11, le 2-chloro-6-éthylamino-4-nitrophénol, le 2-amino-6-chloro-4-nitrophénol, le HC Yellow n° 13, le Basic Blue n° 99, le Basic Brown n° 16, le Basic Brown n° 17, le Basic Red n° 76, le Basic Yellow n° 57 et la 2,6-diamino-3-[(pyridin-3-yl)azo]-pyridine ainsi que leurs sels, et dans laquelle les colorants par oxydation sont choisis parmi le 2,5-diamino-toluène, le 2,4-diaminophénoxyéthanol, la résorcine, la 2-méthylrésorcine, le m-aminophénol, le 4-amino-3-méthyl-phénol, le 4-amino-2-hydroxy-toluène, le 6-amino-3-méthyl-phénol, le 2-amino-4-hydroxyéthylaminoanisol, le 1-naphtol, l'hydroxyéthyl-3,4-méthylènedioxyaniline, le 2,5-diamino-phényléthanol, la N,N-bis(2-hydroxyéthyl)-p-phénylène-diamine, la phényl-méthyl-pyrazolone et le 1-hydroxyéthyl-4,5-diamino-pyrazol ou leurs sels.
